# EUROPEAN PATENT APPLICATION

(11) **EP 4 023 216 A1**
(43) Date of publication of application: **06.07.2022**
(21) Application number: 20877793.8
(22) Date of filing: 16.10.2020
(51) Int. Cl.: A61K 9/24, A61K 31/4439, A61K 33/08, A61K 9/20, A61P 1/04

(54) **PHARMACEUTICAL COMPOSITION COMPRISING PROTON PUMP INHIBITOR AND ANTACID**

(30) Priority: 17.10.2019 KR 20190129323
(71) Applicant: Hanmi Pharm. Co., Ltd., Hwaseong-si, Gyeonggi-do 18536 (KR)
(72) Inventor: CHO, Hyuk Jun, Suwon-si, Gyeonggi-do 16358 (KR); KIM, Bo Sik, Sejong 30049 (KR); IM, Ho Taek, Yongin-si, Gyeonggi-do 16909 (KR); KIM, Yong Il, Gwacheon-si, Gyeonggi-do 13835 (KR)
(74) Representative: Brevalex
(86) International application number: PCT/KR2020/014201
(87) International publication number: WO 2021/075926

(57) **Abstract**

One aspect provides a pharmaceutical composition and a pharmaceutical combination formulation comprising the same, the pharmaceutical composition comprising as active ingredients: a proton pump inhibitor or a pharmaceutically acceptable salt thereof; and an antacid selected from magnesium hydroxide, magnesium oxide, or a mixture thereof.

## Description

### TECHNICAL FIELD

The present disclosure relates to a pharmaceutical composition comprising a proton pump inhibitor and an antacid, or more specifically, to a pharmaceutical composition capable of preventing decomposition of a proton pump inhibitor in the stomach without an enteric coating and improving stability.

### BACKGROUND ART

A proton pump inhibitor is a drug that inhibits the proton pump (H+ / K+-ATPase) of parietal cells to suppress the production of hydrochloric acid, and weakens acidity in the digestive system, thereby showing medicinal effects on dyspepsia, gastroesophageal reflux disease, laryngopharyngeal reflux disease, or digestive ulcer disease. In particular, benzimidazole-based compounds or salts thereof are used as therapeutic agents for digestive ulcers acting as proton pump inhibitors, examples of which are omeprazole, lansoprazole, rabeprazole, pantoprazole, and esomeprazole.

However, such proton pump inhibitors have a drawback in that they easily disassemble or deform under acidic conditions. For example, lansoprazole has poor solubility in water, is very unstable in acid, and easily disassembles in gastric juice, which is an acidic solution, and does not exhibit the desired pharmacological effect. Also, the free-base of esomeprazole has poor physical properties and storage stability, particularly showing a significantly lower stability in acidic and neutral environments than in an alkaline environment. Also, the drug stability of esomeprazole magnesium salt is highly influenced by additives or manufacturing methods, and in particular, the stability in acidic environments is not good.

Enteric formulations have been studied as formulations for improving the stability of the benzimidazole-based proton pump inhibitors, and work by introducing an enteric coating layer to prevent a drug from decomposition by preventing exposure to gastric acid in the stomach, and to dissolve it in the intestine for absorption. For example, Korean Patent Publication No. 10-2008-0005575 presents a method of coating with an enteric polymer to improve the stability of a benzimidazole-based proton pump inhibitor.

However, when a drug is manufactured in this way, a relatively large amount of enteric polymer or an excessive amount of additive is required, and if the enteric coating layer is lost, the drug is easily exposed to gastric acid and cannot be prevented from decomposition, and here is a disadvantage in that the onset time of drug efficacy is delayed. Therefore, it is necessary to develop a formulation that improves the stability of proton pump inhibitors in a gastric acid environment, and provides a rapid onset of the drug efficacy.

### DESCRIPTION OF EMBODIMENTS

### TECHNICAL PROBLEM

An aspect is to provide a pharmaceutical composition that prevents decomposition of proton pump inhibitors in the stomach, improves stability, exhibits a rapid onset of a drug efficacy, and can reduce the amount of additives included in the formulation. The pharmaceutical composition comprises: a proton pump inhibitor or a pharmaceutically acceptable salt thereof; and an antacid selected from magnesium hydroxide, magnesium oxide, and a mixture thereof.

Another aspect is to provide a pharmaceutical combination formulation comprising: a primary layer comprising a proton pump inhibitor or a pharmaceutically acceptable salt thereof; and
a secondary layer comprising one selected from magnesium hydroxide, magnesium oxide, and a mixture thereof.

### SOLUTION TO PROBLEM

An aspect provides a pharmaceutical composition comprising as active ingredients: a proton pump inhibitor or a pharmaceutically acceptable salt thereof; and an antacid selected from magnesium hydroxide, magnesium oxide, and a mixture thereof.

Another aspect provides a pharmaceutical combination formulation comprising:
a primary layer comprising a proton pump inhibitor or a pharmaceutically acceptable salt thereof; and
a secondary layer comprising one selected from magnesium hydroxide, magnesium oxide, and a mixture thereof.

### ADVANTAGEOUS EFFECTS OF DISCLOSURE

The pharmaceutical composition according to an aspect can improve the stability of a proton pump inhibitor or a pharmaceutically acceptable salt thereof, and exhibit a rapid onset of the drug efficacy (shortens Tmax, or the time to reach the maximum concentration (Cmax)). In addition, unlike the commercially available enteric formulation including proton pump inhibitors, the pharmaceutical composition does not include an enteric coating, but an antacid neutralizes the gastric acid and prevents decomposition of the proton pump inhibitors, and the proton pump inhibitors can be rapidly absorbed to promptly exhibit the medicinal effect. Furthermore, the pharmaceutical composition exhibits an area under the blood level curve (AUC) equivalent to that of the commercially available enteric formulation.

Moreover, the pharmaceutical composition according to an aspect includes an antacid that can reduce an amount of additives included in the formulation, and thus minimizes side effects such as acid rebound due to antacids, abdominal bloating due to gas in the gastrointestinal tract, and aerophagia; also, the total weight of the formulation can be minimized for a patient to take the drug more conveniently.

### BRIEF DESCRIPTION OF DRAWINGS

FIG. 1 is a graph showing dissolution rates (%) of esomeprazole according to a dissolution time (min) of formulations prepared according to Examples 1 and 5, and Comparative Examples 1 and 3.
FIG. 2 is a graph showing dissolution rates (%) of esomeprazole according to a dissolution time (min) of formulations prepared according to Examples 4 and 6, and Comparative Examples 2 and 4.
FIG. 3 is a graph showing dissolution rates (%) of esomeprazole according to a dissolution time (min) of formulations prepared according to Examples 1 to 4, and Comparative Examples 5 to 6.
FIG. 4 is a graph showing dissolution rates (%) of esomeprazole according to a dissolution time (min) of formulations prepared according to Examples 5 to 7, and Comparative Examples 7 to 8.
FIG. 5 is a graph showing dissolution rates (%) of esomeprazole or lansoprazole according to a dissolution time (min) of formulations prepared according to Example 4, and Examples 7 to 9.
FIG. 6 is a graph showing evaluation results for pharmacokinetic parameters of esomeprazole as plasma levels (ng/mL), the esomeprazole being included formulations prepared according to Examples 2 and 4 and Comparative Example 9.

### MODE OF DISCLOSURE

Hereinafter, the present disclosure will be described in more detail.

Unless defined otherwise, all technical terms used herein have the same meanings as those generally understood by one of ordinary skill in the art to which the present disclosure belongs. Further, although methods or samples are described herein, those similar or equivalent thereto are also incorporated in the scope of the present disclosure. The contents of all the publications disclosed as references herein are incorporated in the present disclosure.

An aspect provides a pharmaceutical composition comprising as active ingredients: a proton pump inhibitor or a pharmaceutically acceptable salt thereof; and an antacid selected from magnesium hydroxide, magnesium oxide, and a mixture thereof.

The term "proton pump inhibitor (PPI)" as used herein means all drugs that have a pharmacological activity as an inhibitor of H+-K+ ATP-ase (proton pump), and includes all forms, such as a salt, an ester, an amide, an enantiomer, an isomer, a tautomer, a precursor, a derivative, and the like of a known PPI. The final step of gastric acid secretion in the body is regulated by a H+-K+ ATP-ase (proton pump) which releases it into the gastric duct with uptakes of K ions from the gastric duct. A proton pump inhibitor inhibits this pump and strongly hinders the gastric acid secretion.

In an embodiment, the proton pump inhibitor may be one selected from the group consisting of esomeprazole, omeprazole, lansoprazole, rabeprazole, pantoprazole, and combinations thereof. Esomeprazole ((S)-5-methoxy-2-[(4-methoxy-3,5-dimethylpyridin-2-yl) methylsulfinyl]-3H-benzimidazole) is a (S)-optical isomer of omeprazole and is known to have excellent safety and efficacy between the two optical isomers of omeprazole. Lansoprazole (2-{[3-methyl-4-(2,2,2-trifluoroethoxy) pyridine-2-yl] methansulfinyl-1H-1,3-benzothiazole} is commercially available under the product name of Lanston.

The pharmaceutically acceptable salt of the proton pump inhibitor may be any pharmaceutically acceptable salt that can be conventionally used in the art, and may be, for example, a metal salt such as a magnesium (Mg) salt, a strontium (Sr) salt, a lithium salt, a sodium salt, a potassium salt, a calcium salt, or an ammonium salt, but is not limited thereto.

In an embodiment, the proton pump inhibitor or a pharmaceutically acceptable salt thereof may be esomeprazole magnesium salt or lansoprazole.

In addition, the proton pump inhibitor or a pharmaceutically acceptable salt thereof may be used in a form of an anhydride or a hydrate, such as monohydrate, a dihydrate, or a trihydrate. For example, it can be provided as esomeprazole magnesium trihydrate.

As used herein, the term "antacid" or "antacid agent" indicates a compound capable of reducing heartburn (or pyrosis), a typical symptom of an acid over-secretion. Moreover, an antacid is a drug that acts either directly by buffering the pH of the gastric mucosa against excessive gastric acid or gastroesophageal reflux, or indirectly by suppressing the stomach from secreting acid. For example, an antacid may include a substance that indirectly inhibits the acid-secretion in the stomach, or a substance that reduces all the above-mentioned symptoms due to neutralization effect of gastric acidity. In an embodiment, the antacid may be magnesium hydroxide, magnesium oxide, sodium hydrogen carbonate, potassium carbonate, or a mixture thereof, and may preferably be magnesium hydroxide, magnesium oxide, or a mixture thereof.

The pharmaceutical composition may include, with respect to 1 part by weight of a proton pump inhibitor or a pharmaceutically acceptable salt thereof as a proton pump inhibitor, an antacid in an amount of about 4 parts by weight or more, about 5 parts by weight or more, about 5.5 parts by weight or more, about 6 parts by weight or more, about 6.25 parts by weight or more, or about 6.5 parts by weight or more. The pharmaceutical composition may include, with respect to 1 part by weight of a proton pump inhibitor or a pharmaceutically acceptable salt thereof as a proton pump inhibitor, an antacid in an amount of about 25 parts by weight or less, about 20 parts by weight or less, about 18 parts by weight or less, about 17.5 parts by weight or less, about 15 parts by weight or less, about 13 parts by weight or less, about 12.5 parts by weight or less, about 12 parts by weight or less, about 11.5 parts by weight or less, or about 10 parts by weight or less.

The antacid in the pharmaceutical composition may be included in a range with an upper limit and a lower limit with respect to 1 part by weight of the proton pump inhibitor.

For example, the pharmaceutical composition may include, with respect to 1 part by weight of a proton pump inhibitor or a pharmaceutically acceptable salt thereof as a proton pump inhibitor, an antacid in an amount of about 5 parts by weight to about 25 parts by weight, about 5 parts by weight to about 20 parts by weight, about 5 parts by weight to about 17.5 parts by weight, about 6.25 parts by weight to about 20 parts by weight, about 5 parts by weight to about 15 parts by weight, about 5 parts by weight to about 12.5 parts by weight, about 6 parts by weight to about 12 parts by weight, about 6.25 parts by weight to about 11.5 parts by weight, or about 6.25 parts by weight to about 12.5 parts by weight.

The pharmaceutical composition according to an embodiment may include, with respect to 1 part by weight of proton pump inhibitor or a pharmaceutically acceptable salt thereof as a proton pump inhibitor, magnesium hydroxide in an amount of about 6.25 parts by weight or more. The pharmaceutical composition according to an embodiment may include, with respect to 1 part by weight of proton pump inhibitor or a pharmaceutically acceptable salt thereof as a proton pump inhibitor, magnesium oxide in an amount of about 5 parts by weight or more.

The pharmaceutical composition according to an embodiment including esomeprazole or a pharmaceutically acceptable salt thereof, or lansoprazole or a pharmaceutically acceptable salt thereof may include, with respect to 1 part by weight of esomeprazole or lansoprazole, an antacid in an amount of about 5 parts by weight to about 25 parts by weight, about 5 parts by weight to about 20 parts by weight, about 5 parts by weight to about 17.5 parts by weight, about 6.25 parts by weight to about 20 parts by weight, about 4.5 parts by weight to about 15 parts by weight, about 5 parts by weight to about 15 parts by weight, about 6 parts by weight to about 13 parts by weight, or about 6.25 parts by weight to about 12.5 parts by weight. In an embodiment, the antacid may be magnesium hydroxide, magnesium oxide, or a mixture thereof.

The pharmaceutical composition according to an embodiment including esomeprazole or a pharmaceutically acceptable salt thereof as esomeprazole, with respect to 1 part by weight of esomeprazole, may include magnesium hydroxide in an amount of about 5 parts by weight to about 25 parts by weight, about 5 parts by weight to about 20 parts by weight, about 5 parts by weight to about 17.5 parts by weight, about 6.25 parts by weight to about 25 parts by weight, about 6.25 parts by weight to about 20 parts by weight, about 5 parts by weight to about 13 parts by weight, about 6 parts by weight to about 12.5 parts by weight, or about 6.25 parts by weight to about 12.5 parts by weight.

The pharmaceutical composition according to an embodiment including esomeprazole or a pharmaceutically acceptable salt thereof as esomeprazole, with respect to 1 part by weight of esomeprazole, may include magnesium oxide in an amount of about 4 parts by weight to about 25 parts by weight, about 5 parts by weight to about 20 parts by weight, about 5 parts by weight to about 17.5 parts by weight, about 5 parts by weight to about 15 parts by weight, about 4 parts by weight to about 13 parts by weight, about 4.9 parts by weight to about 12 parts by weight, or about 5 parts by weight to about 10 parts by weight.

The pharmaceutical composition according to an embodiment including lansoprazole or a pharmaceutically acceptable salt thereof as lansoprazole, with respect to 1 part by weight of lansoprazole, may include magnesium hydroxide in an amount of about 5 parts by weight to about 25 parts by weight, about 5 parts by weight to about 20 parts by weight, about 5 parts by weight to about 17.5 parts by weight, about 6.25 parts by weight to about 20 parts by weight, about 5 parts by weight to about 13 parts by weight, about 6 parts by weight to about 12.5 parts by weight, or about 6.25 parts by weight to about 12.5 parts by weight.

The pharmaceutical composition according to an embodiment including lansoprazole or a pharmaceutically acceptable salt thereof as lansoprazole, with respect to 1 part by weight of lansoprazole, may include magnesium oxide in an amount of about 4 parts by weight to about 25 parts by weight, about 5 parts by weight to about 20 parts by weight, about 5 parts by weight to about 17.5 parts by weight, about 5 parts by weight to about 15 parts by weight, about 4 parts by weight to about 13 parts by weight, about 4.9 parts by weight to about 12 parts by weight, or about 5 parts by weight to about 10 parts by weight.

The pharmaceutical composition according to an embodiment may include, with respect to the total weight of the formulation, a proton pump inhibitor or a pharmaceutically acceptable salt thereof, in an amount of about 1 wt% to about 30 wt%, about 1 wt% to about 20 wt%, or about 1 wt% to about 10 wt%.

The pharmaceutical composition according to an embodiment may include, with respect to the total weight of the formulation, an antacid selected from magnesium hydroxide, magnesium oxide, and a mixture thereof, in an amount of about 1 wt% to about 70 wt%, about 10 wt% to about 68 wt%, about 10 wt% to about 67 wt%, or about 20 wt% to about 65 wt%.

The pharmaceutical composition according to an embodiment may include: esomeprazole or a pharmaceutically acceptable salt thereof in an amount of about 20 mg to about 40 mg based on the weight of esomeprazole; and
magnesium hydroxide in an amount of about 250 mg or more, or magnesium oxide in an amount of about 200 mg or more.

The pharmaceutical composition according to an embodiment may include esomeprazole or a pharmaceutically acceptable salt thereof in an amount of about 20 mg to about 40 mg based on the weight of esomeprazole; and
magnesium hydroxide in an amount of about 250 mg to about 500 mg or magnesium oxide in an amount of about 200 mg to about 400 mg.

As an antacid of the amount that satisfies the above range is included, it improves the stability of a proton pump inhibitor or a pharmaceutically acceptable salt thereof by preventing it from decomposing in a gastric acid environment, exhibits an area under the blood level curve (AUC) equivalent to that of the commercially available enteric formulation, and at the same time may exhibit a rapid onset of the drug efficacy (shortens Tmax). In addition, the total weight of the formulation can be reduced to increase the convenience in drug-taking.

The pharmaceutical composition according to an embodiment may further comprise a diluent, a binder, a disintegrant, a lubricant, or a combination thereof.

The diluent increases the volume of the formulation, and may be one or more selected from the group consisting of microcrystalline cellulose, lactose, dextrin, mannitol, sorbitol, starch, calcium hydrogen phosphate, anhydrous calcium hydrogen phosphate, calcium carbonate, saccharides, and mixtures thereof, but is not limited thereto. The amount of the diluent may be, with respect to the total weight of the formulation, about 1 wt% to about 50 wt%, about 10 wt% to about 40 wt %, about 20 wt% to about 30 wt%, or about 20 wt% to about 45 wt %.

The binder may be at least one selected from the group consisting of hydroxypropyl cellulose, hydroxypropyl methylcellulose, polyvinylpyrrolidone, pregelatinized starch, and a mixture thereof, but is not limited thereto. The amount of the binder may be, with respect to the total weight of the formulation, about 0.05 wt% to about 20 wt%, about 0.1 wt% to about 10 wt %, or about 1.0 wt% to about 5.0 wt%.

The disintegrant is used to absorb moisture to promote the disintegration of the formulation to improve the dissolution of the drug, and may be one or more selected from the group consisting of a croscarmellose sodium, corn starch, crospovidone, polyvinylpyrrolidone (PVP), sodium starch glycolate, low-substituted hydroxypropyl cellulose, pregelatinized starch, alginic acid, sodium alginate, and a mixture thereof, but is not limited thereto. The amount of the disintegrant may be, with respect to the total weight of the formulation, about 0.05 wt% to about 20 wt%, about 0.1 wt% to about 10.0 wt %, or about 1.0 wt% to about 5.0 wt%.

The lubricant improves the fluidity of the granules to increase the filling property to the die which is the lower part of a tablet press, and it reduces the interparticular friction and friction between the granule, the punch which is the upper part of a tablet press, and the die, thus facilitating the compression and emission of tablets, and may be at least one selected from the group consisting of stearic acid, stearate, talc, corn starch, carnauba wax, hard anhydrous silicic acid, magnesium silicate, synthetic aluminum silicate, hydrogenated oil, white wax, titanium oxide, microcrystalline cellulose, macrogol 4000 and 6000, isopropyl myristate, calcium hydrogen phosphate, and mixtures thereof, but is not limited thereto. The amount of the lubricant may be, with respect to the total weight of the formulation, about 0.01 wt% to about 10.0 wt%, about 0.05 wt% to about 5.0 wt %, about 0.1 wt% to about 2.0 wt%, or about 0.1 wt% to about 1.0 wt%.

In addition, an appropriate amount of any pharmaceutical additive that can be conventionally used in the art may be included. For example, at least one selected from the group consisting of a surfactant, an antioxidant, a preservative, a stabilizer, a flavoring agent, a colorant, a solubilizing agent, a pH adjusting agent, a coating agent, and an arbitrary combination thereof, may be further included, but an additive included is not limited thereto.

The pharmaceutical composition according to an embodiment may not include an enteric coating agent. The pharmaceutical composition can prevent decomposition of proton pump inhibitors in the stomach and improve stability without including an enteric coating agent.

Another aspect provides a pharmaceutical combination formulation comprising:
a primary layer comprising a proton pump inhibitor or a pharmaceutically acceptable salt thereof; and
a secondary layer comprising one selected from magnesium hydroxide, magnesium oxide, and a mixture thereof.

The pharmaceutical combination formulation according to an embodiment may further comprise in its primary or secondary layer at least one selected from a diluent, a binder, a disintegrant, a lubricant, and a mixture thereof.

The pharmaceutical combination formulation including esomeprazole or a pharmaceutically acceptable salt thereof, or lansoprazole or a pharmaceutically acceptable salt thereof may include, with respect to 1 part by weight of esomeprazole or lansoprazole, magnesium hydroxide in an amount of about 6.25 parts by weight or more.

The pharmaceutical combination formulation including esomeprazole or a pharmaceutically acceptable salt thereof, or lansoprazole or a pharmaceutically acceptable salt thereof may include, with respect to 1 part by weight of esomeprazole or lansoprazole, magnesium oxide in an amount of about 5 parts by weight or more.

The primary layer of the pharmaceutical combination formulation according to an embodiment may include: with respect to the total weight of the primary layer, a proton pump inhibitor in an amount of about 1 wt% to about 50 wt%, about 1 wt% to about 45 wt%, or about 1 wt% to about 40 wt%;
a diluent in an amount of about 1 wt% to about 70 wt%, about 1 wt% to about 50 wt%, about 1 wt% to about 49 wt%, or about 1 wt% to about 48 wt%;
a binder in an amount of about 0.05 wt% to about 10 wt%, about 0.1 wt% to about 7 wt%, or about 1 wt% to about 5 wt%;
a disintegrant in an amount of about 0.01 wt% to about 10 wt%, about 0.05 wt% to about 5 wt%, or about 0.5 wt% to about 5 wt%; and
a lubricant in an amount of about 0.01 wt% to about 10 wt%, about 0.05 wt% to about 6 wt%, or about 0.5 wt% to about 5 wt%.

The secondary layer of the pharmaceutical combination formulation according to an embodiment may include: with respect to the total weight of the secondary layer, an antacid selected from magnesium hydroxide, magnesium oxide, and a mixture thereof, in an amount of about 1 wt% to about 80 wt%, about 10 wt% to about 75 wt%, about 30 wt% to about 75 wt% or about 30 wt% to about 70 wt%;
a diluent in an amount of about 1 wt% to about 50 wt%, about 10 wt% to about 40 wt%, or about 15 wt% to about 40 wt%;
a binder in an amount of about 0.1 wt% to about 10 wt%, about 0.5 wt% to about 7 wt%, or about 1 wt% to about 5 wt%;
a disintegrant in an amount of about 0.1 wt% to about 10 wt%, about 0.5 wt% to about 5 wt%, or about 1 wt% to about 5 wt%;
a lubricant in an amount of about 0.01 wt% to about 10 wt%, about 0.05 wt% to about 5 wt%, or about 0.5 wt% to about 2 wt%.

The kind of the diluent, the binder, the disintegrant, or the lubricant is the same as in the description of the pharmaceutical composition. The primary or secondary layer of the pharmaceutical combination formulation according to an embodiment may further comprise microcrystalline cellulose, hydroxypropyl cellulose, croscarmellose sodium, sodium stearyl fumarate, or a combination thereof.

The pharmaceutical combination formulation may be a multilayered tablet such as a bilayer or a trilayer tablet, a tablet-in-tablet (cored tablet), or a capsule.

In an embodiment, the combination formulation may be a bilayer tablet. The bilayer tablet according to the embodiment consists of a primary layer comprising a proton pump inhibitor or a pharmaceutically acceptable salt thereof; and a secondary layer comprising one selected from magnesium hydroxide, magnesium oxide, and a mixture thereof.

In the bilayer tablet, an antacid selected from magnesium hydroxide, magnesium oxide, and a mixture thereof is rapidly released to increase pH in the stomach, thereby enabling the release of a proton pump inhibitor or a pharmaceutically acceptable salt thereof in a higher pH environment, and proton pump inhibitors such as esomeprazole or lansoprazole can be prevented from being decomposed.

Contrastively, in the case of a single layer tablet including a proton pump inhibitor or a pharmaceutically acceptable salt thereof, and one selected from magnesium hydroxide, magnesium oxide, and a mixture thereof in the same layer, before pH in the stomach is sufficiently increased, the proton pump inhibitor is released and the decomposition of the proton pump inhibitor such as esomeprazole or lansoprazole cannot be sufficiently prevented.

The pharmaceutical combination formulation may be a tablet or a capsule coated with polyvinyl alcohol (PVA), hydroxypropylmethylcellulose (HPMC), hydroxypropylcellulose (HPC), or a mixture thereof.

The combination formulation may not include an enteric coating layer. The pharmaceutical combination formulation without including an enteric coating layer can prevent decomposition of proton pump inhibitors in the stomach and improve stability.

The bilayer tablet may be manufactured by preparing a primary granular layer and a secondary granular layer and then pressing them with a multilayer tablet press. Alternatively, a tablet-in-tablet may be manufactured by preparing a first granular layer as an inner core tablet with a conventional single tablet press and film-coating it, then pressing with a tablet-in-tablet press with the inner core tablet as the core layer and a secondary layer as an external layer. Alternatively, a capsule may be manufactured by film-coating a primary granular layer, and filling it into the capsule together with a secondary layer by a capsule filling machine. The pharmaceutical combination formulation according an embodiment may be in the form of a bilayer tablet.

According to the paddle method of dissolution method II of the United States Pharmacopeia (USP), the pharmaceutical combination formulation was tested in a solution of pH 1.3 to pH 2.0, under a condition of a number of revolution of 75 rpm and 37±0.5 °C, and the dissolution rate of the proton pump inhibitor was 55 % or more for 60 minutes. The solution of pH 1.3 to pH 2.0 may be 0.1 N hydrochloric acid solution. In an embodiment, the pharmaceutical combination formulation was tested according to the paddle method of dissolution method II of the USP under a condition of a number of revolution of 75 rpm and 37±0.5 °C, with 2 units of combination formulations (2 tablets, if the combination formulation is a tablet) in a mixed solution of 150 ml of 0.1 N HCI and 450 mL of purified water, and the dissolution rate of the proton pump inhibitor was 55 % or more for 60 minutes.

In the present disclosure, the numerical ranges displayed using the expression "to" include the range with the number that comes before "to" as the lower limit and the number that comes after "to" as the upper limit. As used herein, the expression "about" or "approximately" indicates that the value mentioned may vary to some extent. For example, the value may vary in 10 %, 5 %, 2 %, or 1 %. For example, the meaning of "about 5" includes, an arbitrary value between 4.5 and 5.5, between 4.75 and 5.25, between 4.95 and 5.05, or between 4.9 and 5.1

The expressions such as "have", "may have", "include", "may include", "comprise", or "may comprise" indicate the presence of a characteristic (e.g., number, ingredient, or component), and does not exclude the presence of other characteristics.

Hereinafter, the present disclosure will be described in detail with reference to the following examples. However, the following examples are intended to illustrate the present disclosure, and the scope of the present disclosure is not limited thereto.

### [Example]

Examples 1 to 4: Manufacture of bilayer tablet including esomeprazole and magnesium hydroxide.

According to the compositions described in Table 1, a bilayer tablet including esomeprazole as an active ingredient and magnesium hydroxide as an antacid was prepared.

Specifically, esomeprazole was mixed with microcrystalline cellulose, croscarmellose sodium and hydroxypropyl cellulose for 10 minutes. Here, sodium stearyl fumarate was added as a lubricant and mixed for 5 minutes in order to prepare a final mixture of an upper layer portion.

Magnesium hydroxide was mixed for 10 minutes with microcrystalline cellulose, hydroxypropyl cellulose, and croscarmellose sodium. The result was pressed with a roller compressor (TF-1-A60, Freund Vector) under a condition of a hydraulic pressure of 5 MPa, a feeder speed of 5 rpm, and a roller speed of 1 rpm to form a flake, then it was sieved through openings of 0.8 mm and mixed with the lubricant of sodium stearyl fumarate for 5 minutes in order to prepare a final mixture of a lower layer portion.

A bilayer tablet of 15 kp was prepared by pressing the final mixture of the lower layer portion and the final mixture of the upper layer portion with a tablet press.

Specific compositions of the pharmaceutical combination formulations prepared according to Examples 1 to 4 are shown in Table 1 below.

**[Table 1]**

| **Ingredient (unit: mg)** | **Example 1** | **Example 2** | **Example 3** | **Example 4** |
|---|---|---|---|---|
| Esomeprazole magnesium trihydrate (as esomeprazole 40mg) | 44.5 | 44.5 | 44.5 | 44.5 |
| microcrystalline cellulose (PH101) | 48.5 | 48.5 | 48.5 | 48.5 |
| hydroxypropyl cellulose | 5.0 | 5.0 | 5.0 | 5.0 |
| croscarmellose sodium | 1.0 | 1.0 | 1.0 | 1.0 |
| sodium stearyl fumarate | 1.0 | 1.0 | 1.0 | 1.0 |
| Total sum of an upper layer portion | 100.0 | 100.0 | 100.0 | 100.0 |
| **Magnesium hydroxide** | **500.0** | **350.0** | **300.0** | **250.0** |
| microcrystalline cellulose (PH101) | 150.0 | 150.0 | 150.0 | 150.0 |
| hydroxypropyl cellulose | 15.0 | 15.0 | 15.0 | 15.0 |
| croscarmellose sodium | 15.0 | 15.0 | 15.0 | 15.0 |
| sodium stearyl fumarate | 5.0 | 5.0 | 5.0 | 5.0 |
| Total sum of a lower layer portion | 685.0 | 535.0 | 485.0 | 435.0 |
| **Total sum of a tablet** | **785.0** | **635.0** | **585.0** | **535.0** |

Examples 5 to 7: Manufacture of bilayer tablet including esomeprazole and magnesium oxide.

According to the compositions shown in Table 2, a bilayer tablet including esomeprazole as an active ingredient and magnesium oxide as an antacid was prepared. The bilayer tablets of Examples 5 to 7 were prepared in the same manner as in Example 1, and specific compositions of the pharmaceutical combination formulations prepared according to Examples 5 to 7 are shown in Table 2 below.

**[Table 2]**

| **Ingredient (unit: mg)** | **Example 5** | **Example 6** | **Example 7** |
|---|---|---|---|
| Esomeprazole magnesium trihydrate (as esomeprazole 40mg) | 44.5 | 44.5 | 44.5 |
| microcrystalline cellulose (PH101) | 48.5 | 48.5 | 48.5 |
| hydroxypropyl cellulose | 5.0 | 5.0 | 5.0 |
| croscarmellose sodium | 1.0 | 1.0 | 1.0 |
| sodium stearyl fumarate | 1.0 | 1.0 | 1.0 |
| Total sum of an upper layer portion | 100.0 | 100.0 | 100.0 |
| **Magnesium oxide** | **400.0** | **250.0** | **200.0** |
| microcrystalline cellulose (PH101) | 150.0 | 150.0 | 150.0 |
| hydroxypropyl cellulose | 15.0 | 15.0 | 15.0 |
| croscarmellose sodium | 15.0 | 15.0 | 15.0 |
| sodium stearyl fumarate | 5.0 | 5.0 | 5.0 |
| Total sum of a lower layer portion | 585.0 | 435.0 | 385.0 |
| **Total sum of a tablet** | **685.0** | **535.0** | **485.0** |

Examples 8 to 9: Manufacture of bilayer tablet including lansoprazole and magnesium hydroxide or magnesium oxide.

According to the compositions shown in Table 3, a bilayer tablet including lansoprazole as an active ingredient and magnesium hydroxide or magnesium oxide as an antacid was prepared. The bilayer tablets of Examples 8 to 9 were prepared in the same manner as in Example 1, and specific compositions of the pharmaceutical combination formulations prepared according to Examples 8 to 9 are shown in Table 3 below.

**[Table 3]**

| **Ingredient (unit: mg)** | **Example 8** | **Example 9** |
|---|---|---|
| **Lansoprazole** | **40.0** | **40.0** |
| microcrystalline cellulose (PH101) | 53.0 | 53.0 |
| hydroxypropyl cellulose | 5.0 | 5.0 |
| croscarmellose sodium | 1.0 | 1.0 |
| sodium stearyl fumarate | 1.0 | 1.0 |
| Total sum of an upper layer portion | 100.0 | 100.0 |
| **Magnesium hydroxide** | **250.0** | - |
| **Magnesium oxide** | - | **200.0** |
| microcrystalline cellulose (PH101) | 150.0 | 150.0 |
| hydroxypropyl cellulose | 15.0 | 15.0 |
| croscarmellose sodium | 15.0 | 15.0 |
| sodium stearyl fumarate | 5.0 | 5.0 |
| Total sum of a lower layer portion | 435.0 | 385.0 |
| **Total sum of a tablet** | **535.0** | **485.0** |

### Comparative Examples 1 to 4: Manufacture of bilayer tablet including esomeprazole and sodium hydrogen carbonate or calcium carbonate

According to the compositions shown in Table 4, a bilayer tablet including esomeprazole as an active ingredient and sodium hydrogen carbonate or calcium carbonate as an antacid was prepared. The bilayer tablets of Comparative Examples 1 to 4 were prepared in the same manner as in Example 1, and specific compositions of the pharmaceutical combination formulations prepared according to Comparative Examples 1 to 4 are shown in Table 4 below.

**[Table 4]**

| **Ingredient (unit: mg)** | **Comparative Example 1** | **Comparative Example 2** | **Comparative Example 3** | **Comparative Example 4** |
|---|---|---|---|---|
| Esomeprazole magnesium trihydrate (as esomeprazole 40mg) | 44.5 | 44.5 | 44.5 | 44.5 |
| microcrystalline cellulose (PH101) | 48.5 | 48.5 | 48.5 | 48.5 |
| hydroxypropyl cellulose | 5.0 | 5.0 | 5.0 | 5.0 |
| croscarmellose sodium | 1.0 | 1.0 | 1.0 | 1.0 |
| sodium stearyl fumarate | 1.0 | 1.0 | 1.0 | 1.0 |
| Total sum of an upper layer portion | 100.0 | 100.0 | 100.0 | 100.0 |
| **sodium hydrogen** | **800.0** | **250.0** | - | - |
| **carbonate calcium carbonate** | - | - | **600.0** | **250.0** |
| microcrystalline cellulose (PH101) | 150.0 | 150.0 | 150.0 | 150.0 |
| hydroxypropyl cellulose | 15.0 | 15.0 | 15.0 | 15.0 |
| croscarmellose sodium | 15.0 | 15.0 | 15.0 | 15.0 |
| sodium stearyl fumarate | 5.0 | 5.0 | 5.0 | 5.0 |
| Total sum of a lower layer portion | 985.0 | 435.0 | 785.0 | 435.0 |
| **Total sum of a tablet** | **1085.0** | **535.0** | **885.0** | **535.0** |

### Comparative Examples 5 to 8: Manufacture of bilayer tablet including esomeprazole and magnesium hydroxide or magnesium oxide

According to the compositions described in Table 5 with varying amounts of an antacid, a bilayer tablet including esomeprazole as an active ingredient and magnesium hydroxide or magnesium oxide as an antacid was prepared. The bilayer tablets of Comparative Examples 5 to 8 were prepared in the same manner as in Example 1, and specific compositions of the pharmaceutical combination formulations prepared according to Comparative Examples 5 to 8 are shown in Table 4 below.

**[Table 5]**

| **Ingredient (unit: mg)** | **Comparative Example 5** | **Comparative Example 6** | **Comparative Example 7** | **Comparative Example 8** |
|---|---|---|---|---|
| Esomeprazole magnesium trihydrate (as esomeprazole 40mg) | 44.5 | 44.5 | 44.5 | 44.5 |
| microcrystalline cellulose (PH101) | 48.5 | 48.5 | 48.5 | 48.5 |
| hydroxypropyl cellulose | 5.0 | 5.0 | 5.0 | 5.0 |
| croscarmellose sodium | 1.0 | 1.0 | 1.0 | 1.0 |
| sodium stearyl fumarate | 1.0 | 1.0 | 1.0 | 1.0 |
| Total sum of an upper layer portion | 100.0 | 100.0 | 100.0 | 100.0 |
| **Magnesium hydroxide** | **240.0** | **200.0** | - | - |
| **Magnesium oxide** | - | - | **190.0** | **150.0** |
| microcrystalline cellulose (PH101) | 150.0 | 150.0 | 150.0 | 150.0 |
| hydroxypropyl cellulose | 15.0 | 15.0 | 15.0 | 15.0 |
| croscarmellose sodium | 15.0 | 15.0 | 15.0 | 15.0 |
| sodium stearyl fumarate | 5.0 | 5.0 | 5.0 | 5.0 |
| Total sum of a lower layer portion | 425.0 | 385.0 | 375.0 | 335.0 |
| **Total sum of a tablet** | **525.0** | **485.0** | **475.0** | **435.0** |

### Comparative Example 9: Commercial enteric formulation

Nexium tab 40 mg (40 mg as esomeprazole), a commercially available enteric formulation was chosen as Comparative Example 9.

### Test Example

### Test Example 1: Dissolution test

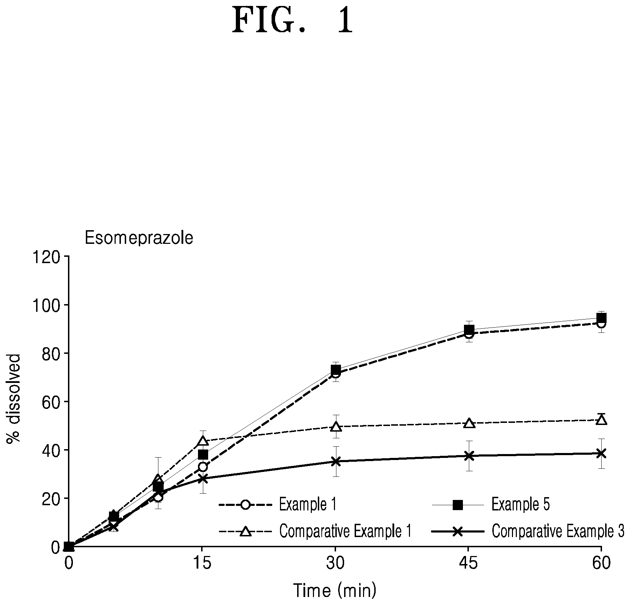

Under the following dissolution conditions and analysis conditions, dissolution rates of esomeprazole of Examples 1 to 9 and Comparative Examples 1 to 8 were measured, and the results are shown in FIGS. 1 to 5.

### <Dissolution Condition>

Eluate: 2 tablets (80 mg as esomeprazole) were tested in a mixed solution of 150 mL of 0.1 N HCI and 450 mL of purified water.
Apparatus: Paddle method of dissolution method II of the (USP, a paddle, 75 ± 2 rpm
Temperature: 37 ± 0.5 °C
Dissolution time: 5, 10, 15, 30, 45, and 60 minutes (after passing the collected eluate through a membrane filter of 0.45 um, it was immediately mixed with 0.25 M of NaOH at a ratio of 5:1)

### - Analysis Conditions -

Column: Inertsil ODS-3V 150 mm x 4.6 mm, 5 µm (column temperature: 25 °C)
Mobile phase: acetonitrile: phosphate buffer solution (pH 7.3): water = 350: 500:150
(wherein the phosphate buffer solution (pH 7.3) is prepared by mixing 10.5 mL of 1 mol/L sodium dihydrogen phosphate and 60.0 mL of 0.5 mol/L sodium hydrogen phosphate and adding water to make 1 L.)
Detector: ultra violet absorptiometer (measurement wavelength 302 nm)
Velocity of flow: 1.0 mL/min
Injection amount: 20 µL
Column temperature: 45 °C

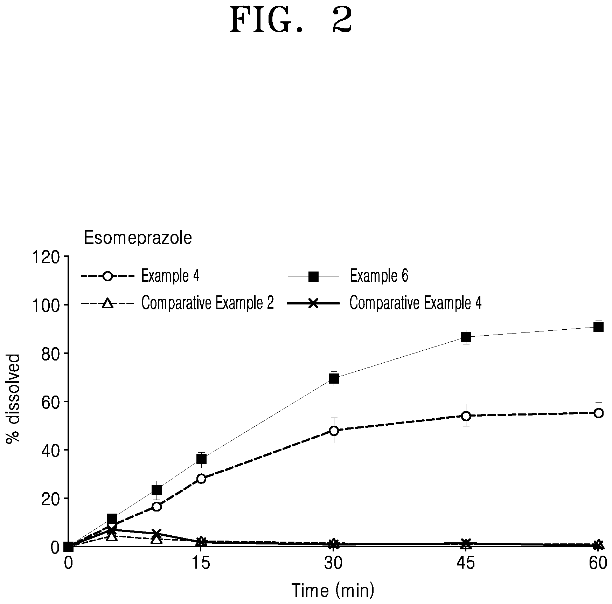

FIGS. 1 and 2 show the dissolution result of esomeprazole according to different types of the antacid. The eluate which is a mixed solution containing 0.1 N HCI and purified water was reproduced with, as the 0.1 N HCI, gastric juice present in the stomach, and as the purified water, water taken with medication, to confirm the decomposition degree of the proton pump inhibitor (PPI), such as esomeprazole, that can be decomposed at a low pH when taken by humans, and the antacid ability of the antacid.

Examples 1 and 5 in FIG. 1 included 500 mg of magnesium hydroxide (12.5 parts by weight per 1 part by weight of esomeprazole) and 400 mg of magnesium oxide (10 parts by weight per 1 part by weight of esomeprazole), respectively, and Comparative Examples 1 and 3 included 800 mg of sodium hydrogen carbonate (20 parts by weight per 1 part by weight of esomeprazole) and 600 mg of calcium carbonate (15 parts by weight per 1 part by weight of esomeprazole), respectively. The dissolution rates of Examples 1 and 5 were higher, indicating that magnesium hydroxide and magnesium oxide have a better decomposition prevention efficacy and antacid power in comparison to sodium hydrogen carbonate and calcium carbonate.

Examples 4 and 6, Comparative Examples 2 and 4 in FIG. 2 included 250 mg of magnesium hydroxide, magnesium oxide, sodium hydrogen carbonate, and calcium carbonate (6.25 parts by weight per 1 part by weight of esomeprazole), respectively. When a comparison was made using the same amount of different antacids, as shown in FIG. 2, it was confirmed that magnesium hydroxide and magnesium oxide of Examples 4 and 6 had better decomposition prevention efficacy and antacid power. In addition, when magnesium hydroxide or magnesium oxide is used as an antacid, the tablet mass can be made smaller compared to when sodium hydrogen carbonate or calcium carbonate is used, thereby providing an improved convenience for a patient.

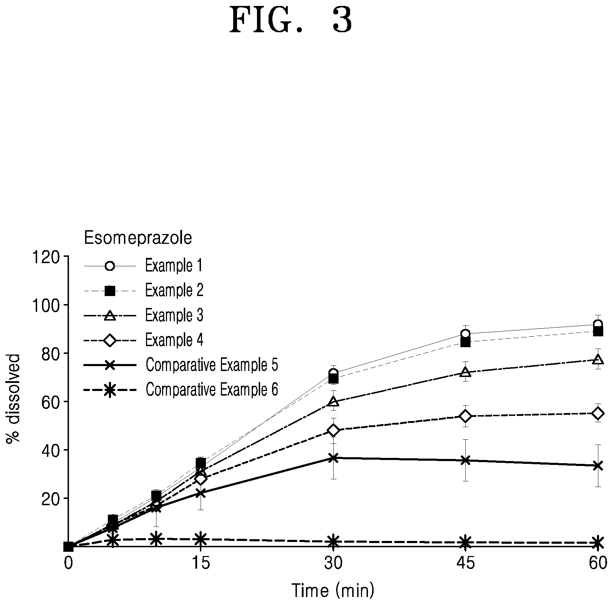

FIG. 3 is a dissolution result of esomeprazole according to different amounts of magnesium hydroxide. As an amount of magnesium hydroxide decreased, a 60-minute dissolution rate of esomeprazole also decreased, when Example 4 and Comparative Example 5 were compared, when an amount of magnesium hydroxide is less than 250 mg, an antacid effect is insufficient, and esomeprazole showed an increased deviation in dissolution, a decreased dissolution rate, and a tendency to decrease dissolution rates over time. Therefore, it is possible to determine that when magnesium hydroxide is used as an antacid, the amount of about 250 mg or more is valid.

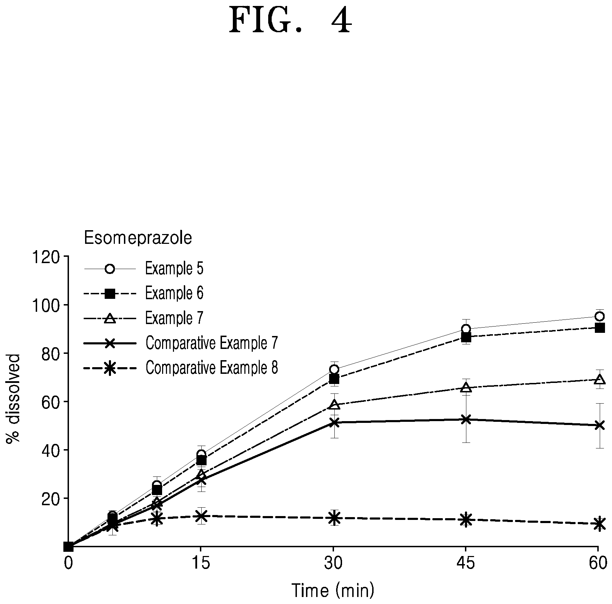

FIG. 4 is a dissolution result of esomeprazole according to different amounts of magnesium oxide. As an amount of magnesium oxide decreased, a 60-minute dissolution rate of esomeprazole also decreased, when Example 7 and Comparative Example 7 were compared, when an amount of magnesium oxide is less than 200 mg, the antacid effect is insufficient, and esomeprazole showed an increased deviation in dissolution, a decreased dissolution rate, and a tendency to decrease dissolution rates over time. Therefore, it is possible to determine that when magnesium oxide is used as an antacid, the amount of about 200 mg or more is valid.

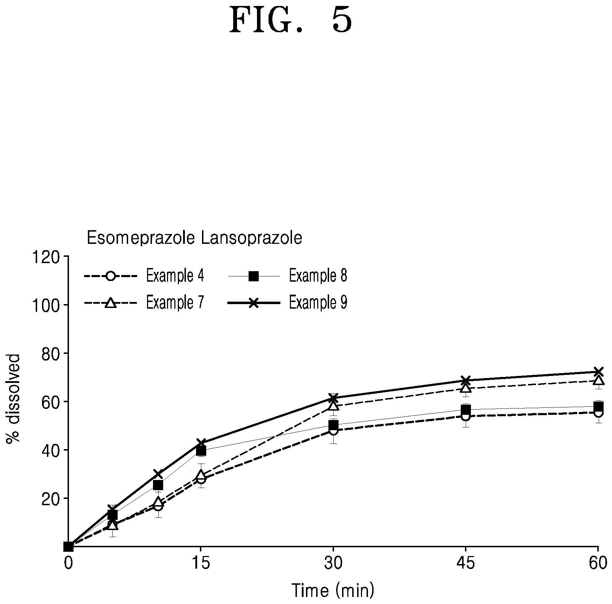

250 mg of magnesium hydroxide or 200 mg of magnesium oxide, of the amounts confirmed to be sufficient for showing an antacid effect in the combination formulation including esomeprazole and magnesium hydroxide or magnesium oxide, were mixed with 40 mg of lansoprazole, a proton pump inhibitor(PPI) apart from esomeprazole, to prepare a combination formulation, and the dissolution results are shown in FIG. 5. When lansoprazole, a widely used PPI drug like esomeprazole, was used, it showed a faster dissolution rate than esomeprazole but the final dissolution rate was similar to that of esomeprazole. Therefore, it is possible to determine that the amount of 250 mg or more of magnesium hydroxide and 200 mg or more of magnesium oxide secures an antacid power, regardless of the PPI drug.

### Test Example 2: Pharmacokinetic evaluation of esomeprazole

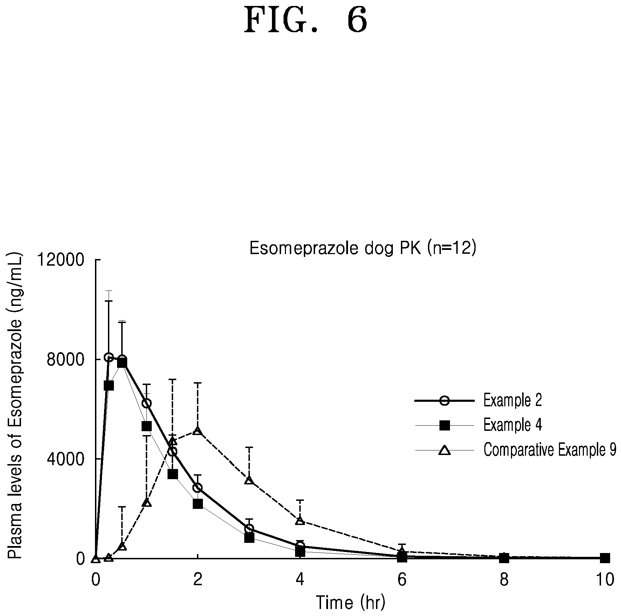

For the compositions of Examples 2 and 4, and Comparative Example 9, the pharmacokinetic parameters of esomeprazole were evaluated with beagles as test subjects. 12 beagles were randomly chosen for a crossover study, and the results are shown in Table 6 and FIG. 6. FIG. 6 is a linear scale showing the arithmetic average plasma levels of esomeprazole (ng/mℓ) per time (hour).

**[Table 6]**

| | **Pharmacokinetic Evaluation of Esomeprazole** | | | | |
|---|---|---|---|---|---|
| **Parameter** | **Example 2 (T1)** | **Example 4 (T2)** | **Comparative Example 9 (R)** | **T1/R** | **T2/R** |
| AUC₀₋₁₀ (ng.hr/mL) | 14442.7±2474.0 | 11563.2±3154.7 | 13645.2±3658.8 | 1.058 | 0.847 |
| Cmax (ng/mL) | 8409.2±1966.1 | 8200.8±1977.6 | 6308.4±1862.4 | 1.333 | 1.300 |
| Tmax (h) | 0.4±0.1 | 0.5±0.2 | 1.8±0.6 | 0.222 | 0.277 |

As shown in Table 6 and FIG. 6, Examples 2 and 4 when compared to Comparative Example 9 (Nexium tab, 40 mg), a commercially available enteric formulation, showed shortened Tmax and an equal level of area under the plasma level curve (AUC). Generally, the inhibitory effect of a proton pump inhibitor on gastric acid secretion is known to be proportional to the absorbed amount (AUC) rather than the maximum concentration (Cmax) or the shape of the blood level graph (J Neurogastroenterol Motil, Vol. 19 No.1 January, 2013). Thus, Examples 2 and 4 shows a medicinal effect equivalent to the commercially available enteric formulation, and at the same time, exhibits a rapid onset of the drug efficacy as can be seen from shortened Tmax. In addition, since Example 4 including 250 mg of magnesium hydroxide showed a 60-minute dissolution rate of about 56 % under the dissolution condition of Test Example 1, it was confirmed that a formulation having a dissolution rate of about 55 % or more under the test condition of Test Example 1 will show an AUC equivalent to that of Nexium tab, a commercially available enteric formulation.

Hereinabove, the present disclosure has been described with reference to preferred examples thereof. It will be understood by those skilled in the art that various changes may be made therein without departing from the spirit and scope of the present disclosure. Therefore, the examples disclosed herein should be considered in an illustrative aspect rather than a restrictive aspect. The scope of the present disclosure is defined by the appended claims rather than by the foregoing description, and all differences within the scope of equivalents thereof should be construed as being included in the present invention.

## Claims

1. A pharmaceutical composition comprising as active ingredients: a proton pump inhibitor or a pharmaceutically acceptable salt thereof; and an antacid selected from magnesium hydroxide, magnesium oxide, and a mixture thereof.

2. The pharmaceutical composition of claim 1, wherein the proton pump inhibitor is one selected from the group consisting of esomeprazole, omeprazole, lansoprazole, rabeprazole, pantoprazole, and combinations thereof.

3. The pharmaceutical composition of claim 1, wherein, in the pharmaceutical composition, magnesium hydroxide in an amount of 6.25 parts by weight or more is included when the proton pump inhibitor or a pharmaceutically acceptable salt thereof is included as a proton pump inhibitor in an amount of 1 part by weight.

4. The pharmaceutical composition of claim 1, wherein, in the pharmaceutical composition, magnesium oxide in an amount of 5 parts by weight or more is included when the proton pump inhibitor or a pharmaceutically acceptable salt thereof is included as a proton pump inhibitor in an amount of 1 part by weight.

5. The pharmaceutical composition of claim 1, wherein the proton pump inhibitor or a pharmaceutically acceptable salt thereof is esomeprazole magnesium salt or lansoprazole.

6. The pharmaceutical composition of claim 2, wherein, in the pharmaceutical composition, magnesium hydroxide in an amount ranging from 6.25 parts by weight to 25 parts by weight of is included when esomeprazole or a pharmaceutically acceptable salt thereof is included as esomeprazole in an amount of 1 part by weight.

7. The pharmaceutical composition of claim 2, wherein, in the pharmaceutical composition, magnesium oxide in an amount ranging from 5 parts by weight to 15 parts by weight of is included when esomeprazole or a pharmaceutically acceptable salt thereof is included as esomeprazole in an amount of 1 part by weight.

8. The pharmaceutical composition of claim 2, wherein the pharmaceutical composition comprises: 20 mg to 40 mg of esomeprazole or a pharmaceutically acceptable salt thereof based on the weight of esomeprazole; and 250 mg to 500 mg of magnesium hydroxide or 200 mg to 400 mg of magnesium oxide.

9. The pharmaceutical composition of claim 1, further comprising a diluent, a binder, a disintegrant, a lubricant, or a combination thereof.

10. The pharmaceutical composition of claim 1, wherein an enteric coating agent is not included.

11. A pharmaceutical combination formulation comprising:
a primary layer comprising a proton pump inhibitor or a pharmaceutically acceptable salt thereof; and
a secondary layer comprising one selected from magnesium hydroxide, magnesium oxide, and a mixture thereof.

12. The pharmaceutical combination formulation of claim 11, wherein the primary layer or the secondary layer further comprises microcrystalline cellulose, hydroxypropyl cellulose, croscarmellose sodium, sodium stearyl fumarate, or a combination thereof.

13. The pharmaceutical combination formulation of claim 11, wherein the combination formulation is a bilayer tablet.

14. The pharmaceutical combination formulation of claim 11, wherein the combination formulation does not include an enteric coating layer.

15. The pharmaceutical combination formulation of claim 11, wherein the combination formulation when tested according to the paddle method of the dissolution method II of the US Pharmacopeia (USP) under a condition of a number of revolution of 75 rpm and 37±0.5 °C, with 2 units of combination formulations in a mixed solution of 150 mL of 0.1 N HCI and 450 mL of purified water, has a dissolution rate of the proton pump inhibitor of 55 % or more for 60 minutes.
